# EUROPÄISCHE PATENTSCHRIFT

(11) **EP 2 767 306 B1**
(45) Veröffentlichungstag und Bekanntmachung des Hinweises auf die Patenterteilung: **11.07.2018**
(21) Anmeldenummer: 14155191.1
(22) Anmeldetag: 14.02.2014
(51) Int. Cl.: A61N 1/05, A61B 17/34, A61B 17/00

(54) **Chirurgisches Applikationswerkzeug zur Implantation eines Elektrodendrahts**
Surgical applicator tool to implant an electrode
Applicateur chirurgical d'implantation de fil d'électrode

(30) Priorität: 14.02.2013 US 201361764592 P; 02.07.2013 DE 202013102904 U
(43) Veröffentlichungstag der Anmeldung: 20.08.2014
(73) Patentinhaber: Possover, Marc, Prof. Dr., 6032 Hagendorn (CH)
(72) Erfinder: Possover, Marc, Prof. Dr., 6032 Hagendorn (CH)
(74) Vertreter: Patentanwälte Behrmann Wagner PartG mbB

(56) Entgegenhaltungen:
- WO-A2-2007/126632
- WO-A2-2011/103530
- US-A1- 2007 173 900
- US-A1- 2008 269 763

## Beschreibung

Die vorliegende Erfindung betrifft ein chirurgisches Applikationswerkzeug nach dem Oberbegriff des Hauptanspruchs. Ferner betrifft die vorliegende Anmeldung einen Elektrodendraht, der insbesondere zum Zusammenwirken mit einem derartigen chirurgischen Applikationswerkzeug durch Einführen in die Hülse dieses Applikationswerkzeugs ausgebildet ist, sowie ein System bestehend aus dem chirurgischen Applikationswerkzeug und dem Elektrodendraht.

Aus dem Stand der Technik sind Methoden sowie Werkzeuge zur Implantation von Elektroden in den menschlichen Körper allgemein bekannt. In diesem allgemeinen Kontext ist es dabei insbesondere als bekannt vorauszusetzen, Elektrodendrähte, also langgestreckte, drahtförmige Leiter mit einends ansitzender Kontaktfläche und anderenends einem Anschluss für eine Signalerzeugungsquelle an oder in unmittelbare Nähe eines Nervs im menschlichen Körper zu implantieren, um mittels der Signalerzeugungsquelle erzeugte elektrische Signale zur Stimulation von Nerven bzw. Nervenenden an diese anzulegen.

So hat der Anmelder, bekannt unter der Kennzeichnung LION, eine laparoskopische Operationstechnologie entwickelt, mit welcher in therapeutisch besonders wirksamer Weise Elektrodendrähte in einen Beckeninnenbereich bzw. Beckenboden eines Patienten implantiert werden können, um dort die Stimulationssignale den pelvinen Nerven, insbesondere den Nervenenden bzw. Nervenwurzeln stimulativ zuzuführen. Für die Durchführung dieser Technologie ist es bekannt, ein Endoskop zu verwenden, wobei ein am Endoskopleiter vorgesehener Arbeitskanal als Schacht dazu dient, unter visueller Kontrolle mittels des Endoskops den vorgesehenen Elektrodendraht (bei geeignet an die Implantationsposition geführtem Endoskopkopf und geeignet chirurgisch aufbereiteter Implantationsposition am Nerv bzw. der pelvinen Nervenwurzel) zu implantieren.

Allerdings ist eine derartige, als gattungsbildend vorausgesetzte Technologie kompliziert in Handhabung und Durchführung: Nicht nur sind beträchtliche Erfordernisse an die operativen Kenntnisse bzw. das chirurgische Vermögen der befassten Bedienperson am Endoskop zu stellen, auch ist die durch die bekannte Technologie bedingte, im Wesentlichen parallele Ausrichtung zwischen optischer Betrachtungsachse einerseits (der visuellen Kontrolle bzw. Kontrollierbarkeit durch das Endoskop) und der Zuführung des Elektrodendrahtes durch den endoskopischen Arbeitsschacht andererseits ungünstig für eine exakte Ausrichtung und Positionierung gerade des kritischen Nervenkontaktabschnitts am Ende des Elektrodendrahts. Mit anderen Worten, eine einfache und positionierungssichere Handhabung des Elektrodendrahts am Implantationsort im menschlichen Beckeninnenbereich unter optischer Kontrolle des Endoskops ist gerade bei orthogonal verlaufenden Geometrien erschwert, was die Anforderungen an den Operateur weiter erhöht.

Ein weiteres Problem bei dieser aus dem Stand der Technik bekannten Vorrichtung besteht darin, dass bei über den Arbeitskanal eines Endoskops implantierter Drahtelektrode diese (nach einem Entfernen des Endoskops, wodurch die Elektrode dann am Implantationsort belassen ist) die Drahtelektrode mit ihrem dem Nervenkontaktabschnitt entgegengesetzten Anschlussabschnitt aus dem für das Endoskop benutzten Körperzugang - typischerweise im Bauchbereich liegend, etwa dem Nabel - herausragt. Um diesen Anschlussabschnitt des Elektrodendrahtes dann mit einer Signalerzeugungsquelle (welche typischerweise ebenfalls unter der Haut des Patienten implantiert wird) zu verbinden, ist ein Verlegen bzw. operatives Verbringen des anschlussseitigen Endes des Elektrodendrahtes im oberflächlichen Körperbereich notwendig, was einen Operationsaufwand zusätzlich erhöht und eine weitere potentielle Belastung des Patienten bringt. Die US 2008/0269763 A1 beschreibt ein chirugisches Werkzeug zur Implantierung einer medizinischen Vorrichtung in einem Patienten. Das Werkzeug umfasst ein Gehäuse, welches von einem Benutzer gehalten wird sowie eine Nadel, welche an das Gehäuse angebracht ist. Die Nadel ist derart ausgestaltet, dass eine Kanüle über ihr positioniert werden und ins Zielgewebe eingebracht werden kann.

Aus der WO 2011103530 A2 ist ein System aus einer Elektrode und einem Werkzeug beschrieben, das ausgebildet ist, die Elektrode während einer Implantation in die gewünschte Position zu bringen.

Aufgabe der vorliegenden Erfindung ist es daher, eine Vorrichtung sowie ein System zu schaffen, mit welchen die medizinisch-therapeutisch bewährte und überaus nutzbringende Implantation von Drahtelektroden insbesondere im Beckeninnenbereich bzw. Beckenbodenbereich des menschlichen Körpers vereinfacht werden kann, damit auch weniger geübten Bedienpersonen zugänglich ist, gleichzeitig die zuverlässige, positionssichere Implantation von Nervenkontaktabschnitten an pelvinen Nerven bzw. Nervenwurzeln auch bei geometrisch winklig zu einer endoskopischen Beobachtungsrichtung verlaufenden Nervengeometrien zuverlässig gestattet. Gleichzeitig ist eine Vorrichtung bzw. ein System zu schaffen, wodurch minimalinvasiv, mit geringem traumatischem bzw. Verletzungsrisiko am Implantationsort bzw. bei der Zuführung zum Implantationsort eine zuverlässige und präzise Elektrodenimplantation erfolgen kann. Schließlich ist das Problem zu lösen, eine einfachere Verleg- und Kontaktierbarkeit des Elektrodendrahtes am dem Nervenkontaktabschnitt entgegengesetzten Ende zu bewirken, insbesondere den Elektrodendraht bereits so vorzusehen, dass dieser an seinem Anschlussabschnitt mit geringem Aufwand, kontaktsicher und ohne die Notwendigkeit komplexer intrakoporaler Verlegevorgänge mit einem Signalerzeuger (welcher weiter bevorzugt ebenfalls implantiert sein soll) kontaktiert werden kann.

Die Aufgabe wird durch das chirurgische Applikationswerkzeug mit den Merkmalen des Hauptanspruchs 1 gelöst. Vorteilhafte Weiterbildungen der Erfindung sind in den jeweiligen Unteransprüchen beschrieben. In erfindungsgemäß vorteilhafter Weise ermöglicht zunächst das chirurgische Applikationswerkzeug in der erfindungsgemäßen Kombination aus Dorn und darüber gesteckter bzw. geführter Hülse das Erreichen der gewünschten Implantationsposition im Beckeninnenbereich, indem von extrakorporal das Werkzeug durch den unteren Bauchbereich des Patienten und dann entlang des Beckeninneren (genauer: Der Beckeninnenwand) bis zu den Beckennerven geführt wird. Damit ermöglicht es die vorliegende Erfindung, im in den Körper eingeführten Zustand des Applikationswerkzeugs und nach dem Entfernen des Dorns (d.h. bei verbleibender und durch den Hülsen-Innenraum eine Führung für den nunmehr von extrakorporal einzusetzenden Elektrodendraht vorgesehenen Hülse) sämtliche relevanten Beckennerven bzw. deren im Beckeninneren gelegene Wurzeln zu erreichen. Zu denen gehören die relativ oberflächigen Nerven, etwa den Plexus lumbalis, Nervus femoralis, Nervus ilio-ingunalis, Nervus genitofemoralis, Nervus cutaneo-femoralis lateralis oder Nervus iliohypogastricus. Gleichermaßen lassen sich durch das erfindungsgemäße Applikationswerkzeug auch die tieferliegenden pelvinen Nerven erreichen, wie den Plexus sacralis, den Ischias-Nerv, den Nervus femoralis, die Nervi splanchnici pelvini, den Nervus pudendus oder den Nervus des levator ani, die pexi hypogastrici superiore und inferiore.

Um das Applikationswerkzeug, ausgehend von einem (etwa im unteren linksseitigen Bauchbereich des Patienten zu bildenden Eintritt) retroperitoneal entlang der Wand der Bauchdecke führen zu können, weist das Applikationswerkzeug im Bereich des Schaftabschnitts zumindest ein bogenförmig gekrümmtes Segment auf, welches insbesondere an die Krümmung des Pelvis angepasst sein kann; auf diese Weise ist in besonders günstiger Weise eine Implantation des Elektrodendrahts an den Ischias-Nerv, den Nervus obturatorius oder den Nervus pudendus (bzw. deren Wurzeln im Beckenboden) vereinfacht; insbesondere kann auf diese Weise die Platzierung des (in der Hülse zu führenden) Elektrodendrahts mit dessen Nervenkontaktabschnitt an den tiefliegenden Sakralwurzeln, medial der iliakalen Gefäße, erfolgen.

Zu diesem Zweck ist es im Rahmen der Erfindung vorgesehen, das mindestens eine bogenförmige Segment des Werkzeugs mit einem Bogen-Biegeradius im Bereich zwischen 15 cm und 70 cm, insbesondere zwischen 20 cm und 40 cm, zu versehen, um insoweit die verschiedenen anatomischen Gegebenheiten (etwa bei Patienten mit entsprechend verschiedener Beckeninnengeometrie) berücksichtigen zu können.

Ergänzend und im Rahmen der Erfindung ist zudem der Dorn (und damit die darauf lösbar geführte Hülse) plastisch biegbar, so dass eine Bedienperson ggf. zusätzliche Anpassungen vornehmen kann. Die zudem im Rahmen der Erfindung vorgesehene bogenförmige Ausgestaltung des Schaftabschnitts schließt zusätzlich ein, auch mehrere Bögen oder verschiedene Abschnitte mit verschiedenen Biegeradien, insbesondere auch einer S-Bogenform, vorzusehen.

Im Rahmen der Erfindung liegt es zudem, den bogenförmig ausgebildeten Abschnitt nicht mit einem konstanten Biegeradius (also kreisbogenförmig) auszugestalten, sondern den Biegeradius entlang des Bogenabschnitts zu verändern, wobei der Biegeradius in Richtung auf die Eingriffsspitze verlängert ist, mit anderen Worten, die Krümmung in Richtung auf die Eingriffsspitze (graduell) flacher ausgestaltet ist, was sich wiederum positiv auf die Einführ- und Handhabbarkeit des Werkzeugs in den besonderen anatomischen Verhältnissen im Beckeninnenbereich auswirkt.

Einer derartigen Anpassung an die anatomisch-geometrischen Verhältnisse im Beckeninnenraum dienen ebenfalls die geraden Segmente des Dorns, welche vorteilhaft entweder griffsseitig dem bogenförmigen Segment benachbart, ergänzend oder alternativ in Richtung auf die Eingriffsspitze dem bogenförmigen Segment benachbart vorgesehen sein können, um insoweit für den Einführungs- und Bewegungs- bzw. Manipulationsprozess bis hin zum Implantationsort eine bestmögliche Anpassung zu erhalten.

Erfindungsgemäß weiterbildend ist das Applikationswerkzeug, insbesondere am Außenmantel der Hülse, glatt ausgestaltet, so dass beim Einführen und Bewegen des Applikationswerkzeugs im Körper keine Reibung mit umliegendem Gewebe bzw. eine minimierte Gefahr von Verletzungen benachbarter Strukturen existiert. Auch bewirkt eine glatte homogene Gestaltung der äußeren Mantelfläche des Applikationswerkzeugs, insbesondere der darauf beim Einbringen aufsitzenden Hülse, eine vereinfachte Dreh- bzw. Rotierbarkeit des Applikators durch die Bauchdecke beim Einführen bzw. nach dem Einführen und nachfolgendem Positionieren des Werkzeugs bis zum nervennahen Implantationsort.

Wiederum vorteilhaft im Rahmen von Weiterbildungen der Erfindung weist das Applikationswerkzeug eingriffsseitig des Dorns eine (weiter bevorzugt austausch- bzw. lösbare) Eingriffsspitze auf, welche im Rahmen einer bevorzugten Ausführungsform der Erfindung abgerundet bzw. stumpfkonusförmig ausgestaltet ist, um beim Einführen eine Verletzung von Nerven bzw. Gefäßen (gleichermaßen auch beim Hindurchtreten durch die Bauchdecke) zu vermeiden, gleiches gilt für das Platzieren der Eingriffsspitze am Implantationsort für den Elektrodendraht. Wiederum bevorzugt im Rahmen der Erfindung ist es, eine derartige abgerundete oder konischstumpf zulaufende Spitze (weiter bevorzugt schraubbar) auszutauschen gegen ein spitz zulaufendes Eingriffsspitzenelement am eingriffsseitigen Ende des Dorns; eine derartige Konfiguration der Erfindung wird dann vorteilhaft genutzt werden können, um im Bauchdeckenbereich (unterhalb der Haut) eine Platzierung und Verlegung weiterbildungsgemäß vorgesehener Verbindungs-Kabelmittel zu ermöglichen; diese Verbindungs-Kabelmittel (als elektrisches Verbindungskabel zwischen dem Anschlussabschnitt des Elektrodendrahts und einem vorteilhaft ebenfalls im Bauchdeckenbereich zu implantierenden Signalerzeuger) ermöglichen es vorteilhaft, diesen Signalerzeuger (etwa realisiert in der Art einer Schrittmacher-Elektronik) entfernt vom Bauchdeckeneintritt für das erfindungsgemäße Applikationswerkzeug zu platzieren, mit dem weiterbildungsgemäßen Vorteil, mögliche Infektionsgefahr zu vermindern.

Im Rahmen der Erfindung liegt es ferner, den erfindungsgemäßen Griffabschnitt des Dorns (über welchen sich üblicherweise die erfindungsgemäße Hülse nicht erstreckt) zur Vereinfachung der Einführ- und Manipulierbarkeit in den/im Körper gebogen bzw. gewinkelt auszugestalten, da auf diese Weise, weiter bevorzugt ohne eine separate Baugruppe außerhalb des Dorns, dieser sowohl entlang seiner Erstreckungsrichtung einführbar ist, als auch nach dem Einführen, durch extrakorporale Manipulation am Griffabschnitt, geeignet gedreht, bzw., in der Art einer Schwenk-Drehbewegung, entlang der Beckeninnenwand zum Implantationsort geführt werden kann.

Die erfindungsgemäß dem Dorn zur Realisierung des Applikationswerkzeugs vorgesehene Hülse ist zunächst erfindungsgemäß vorteilhaft so ausgestaltet, dass diese einerseits (während des erfindungsgemäßen Einbringens des Werkzeugs in den Körper und an den Implantationsort) auf dem Dorn sitzt, in der eingesetzten Position dann jedoch das Wieder-Herausführen des Dorns gestattet, ohne dass sich wesentlich die Position (und Form) der Hülse verändert. Diese bietet damit dann durch ihren (nach Entfernen des Dorns freien) Hülsen-Innenraum eine Führung für das nachfolgende Einbringen des Elektrodendrahts an, wobei dieser Elektrodendraht automatisch und ohne die Notwendigkeit weiterer komplexer Manipulation durch das endseitige Einführen in die Hülse an seine Implantation am Nerv gebracht werden kann.

Erfindungsgemäß vorteilhaft ist damit diese Hülse aus einem (plastisch) deformierbaren Material, etwa einem Kunststoff- und/oder Metallmaterial hergestellt, um sich geeignet an die bogenförmige Ausgestaltung des Dorn-Schaftabschnitts anpassen zu können, wobei, nach dem Entfernen des Dorns, die vorteilhafte Biegesteifigkeit der Hülse nach dem Entfernen des Dorns die Hülsenform im Körper beibehält. Die Erfindung sieht zudem vor, die Hülse aus einem (zumindest abschnittsweise) transparenten oder teiltransparenten Material so auszugestalten, dass, etwa mittels endoskopischer Überwachung, beim Einführen des Elektrodendrahts dessen Progression durch die Hülse bis zum Implantationsort verfolgt werden kann.

Wie auch die vorteilhaft runde bzw. stumpfe Ausgestaltung der Eingriffsspitze des Dorns ist entsprechend auch das eingriffsseitige (distale) Ende der Hülse geeignet gerundet bzw. stumpf ausgebildet, wieder mit dem Vorteil, etwaige Verletzungen oder Rupturen an Geweben, Nerven oder Gefäßen zu verhindern bzw. zu minimieren.

Der eingesetzte Elektrodendraht kann zum einen als gängige, ansonsten als aus dem Stand der Technik bekannt vorauszusetzende Drahtelektrode realisiert sein, welche in üblicher Weise am distalen Ende einen ein- oder mehrpoligen Nervenkontaktabschnitt aufweist, welcher (entsprechend ein- oder mehrpolige) mantelseitige Kontaktflächen ausbildet, welche geeignete litzenartige und voneinander isolierte Innenleiter des (typischerweise aus einem biegsamen Kunststoffmaterial realisierten) Leitungsdrahtes bzw. Leitungsabschnitts kontaktieren. Wiederum in gattungsgemäß bekannter Weise sind diese litzenartigen Innenleiter, weiter bevorzugt über standardisierte Verbinder bzw. Schnittstellen, elektrisch und mechanisch kontaktierbar mit einem Signalerzeuger, welcher in ansonsten bekannter Weise die mantelseitigen Kontaktflächen mit elektrischen Stimulationsimpulsen für die zu behandelnden Nerven versieht. Weiter weist nunmehr dieser Elektrodendraht mantelseitig vorgesehe Widerhaken- und/oder Verriegelungsmittel auf, welche bevorzugt im Bereich des Nervenkontaktabschnitts vorgesehen werden und so ausgestaltet sind, dass einerseits, während des Einfügens und Bewegens durch die Hülse, ein leichtes bidirektionales Gleiten entlang des Hülsen-Innenraums ermöglich ist. Sobald jedoch distalendseitig der Elektrodendraht mit seinem Nervenkontaktabschnitt aus der Hülse heraustritt, greifen die dort bevorzugt ausgebildeten Widerhaken- bzw. Verriegelungsmittel in umgebendes Gewebe und sorgen dort in der Art eines Widerhakens für ein (zumindest im Hinblick auf einen erhöhten notwendigen Kraftaufwand für ein Zurückziehen) Verriegeln am Implantations- bzw. Eingriffsort.

Genauer gesagt sind diese Widerhaken- bzw. Verriegelungsmittel als Spreizvorrichtung schuppenartig bzw. blatt- bzw. flügelartig vom Mantel geneigt zur radialen Richtung (insbesondere flach) abstehend ausgebildet, so dass ein Gleiten bzw. Bewegen auch im Gewebe entlang einer Einführungs- und Vorschubrichtung (d.h. in der Richtung des Nervenkontaktabschnitts) ermöglicht ist, ein Zurückziehen bzw. Gleiten jedoch in der Gegenrichtung, d.h. in der Richtung des Anschlussabschnitts, zu einer Aufspreizung der Widerhaken- bzw. Verriegelungsmittel und mithin zu der gewünschten Sperr- bzw. Verriegelungswirkung führt.

In der konkreten Ausgestaltung ist diese Sperr- bzw. Verriegelungswirkung so ausgestaltet, dass, bei erhöhtem Krafteinsatz, nach wie vor ein Zurück- bzw. Herausziehen ermöglicht ist (und ohne dass signifikante Verletzungen bzw. Beschädigungen von Gewebe bzw. Gefäßen zu befürchten sind), allerdings ist der Kraftaufwand für ein derartiges Zurückbewegen erhöht, was die vorteilhafte Wirkung hat, dass nicht nur während des Implantationsprozesses ein unbeabsichtigtes Herausziehen des Elektrodendrahtes erschwert ist, auch führen Relativbewegungen im Körper zwischen den Gewebeabschnitten entlang der Elektrode nicht zu Rückzieh- bzw. Rückholbewegungen; diese werden vielmehr wirksam von den Widerhaken- bzw. Verriegelungsmitteln verhindert. Ein weiterer Elektrodendraht ist mantelseitig, bevorzugt außerhalb der Widerhaken- bzw. Verriegelmittel, mit einer (durchgehenden, punktuellen oder abschnittsweisen) visuell ablesbaren oder abtastbaren Markierung versehen, welche es auch bei lediglich einem freigelegten Abschnitt dieses Elektrodendrahtes ermöglicht festzustellen, an welchem Ende bzw. auf welcher Seite eines betrachteten Leitungsstücks der Nervenkontaktabschnitt, und wo entsprechend der gegenüberliegende Anschlussabschnitt liegt; eine derartige vorteilhafte Weiterbildung hat den Vorteil, dass im Wege von weiteren Maßnahmen an einem implantierten Elektrodendraht keine versehentliche Extraktion erfolgt, wenn ein befasster Operateur (ohne die vollständige Elektrode freigelegt zu haben) nicht den Nervenkontaktabschnitt bzw. dessen Relativlage erkennen kann. Ein beispielhaftes System bringt das erfindungsgemäß chirurgische Applikationswerkzeug zusammen mit dem Elektrodendraht, wobei zusätzlich dieses System noch um die vorstehend beschriebene Verlängerungs-Kabelmittel und/oder die Signalerzeugungsmittel ergänzt werden kann, so dass mit der vorliegenden Erfindung in umfassender Weise die Erzeugnisse bereitstehen, mit welchen die nutzbringende Implantation von Elektroden zur Stimulation von Nerven im Beckeninnenbereich vereinfacht und damit einer größeren Zielgruppe nutzbringend zugänglich gemacht werden kann.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Fig. 1:: eine Seitendarstellung des erfindungsgemäßen Dorns als Komponente des chirurgischen Applikationswerkzeugs gemäß einer ersten Ausführungsform;
- Fig. 2:: eine Schemadarstellung der zusammen mit dem Dorn der Fig. 1 zu verwendenden Hülse;
- Fig. 3:: eine Schemadarstellung des zusammengeführten Applikationswerkzeugs aus Dorn und Hülse gemäß dem ersten Ausführungsbeispiel der Fig. 1, 2;
- Fig. 4:: eine Schemaansicht des Elektrodendrahts gemäß einer weiteren Ausführungsform, insbesondere zur Verwendung mit dem Ausführungsbeispiel der Fig. 1 bis 3 und zwei verschiedene Varianten zur Ausbildung der Widerhaken- bzw. Verriegelungsmittel;
- Fig. 5:: eine Detailansicht der Eingriffspitze des Applikationswerkzeugs der Fig. 1, 2 im montierten Zustand und
- Fig. 6:: ein Überblickschaubild zum Verdeutlichen einer typischen Verbrauchs- bzw. Benutzungsverpackung für das beispielhafte System zur chirurgischen Implantation eines Elektrodendrahts in einem Beckeninnenbereich eines menschlichen Körpers.

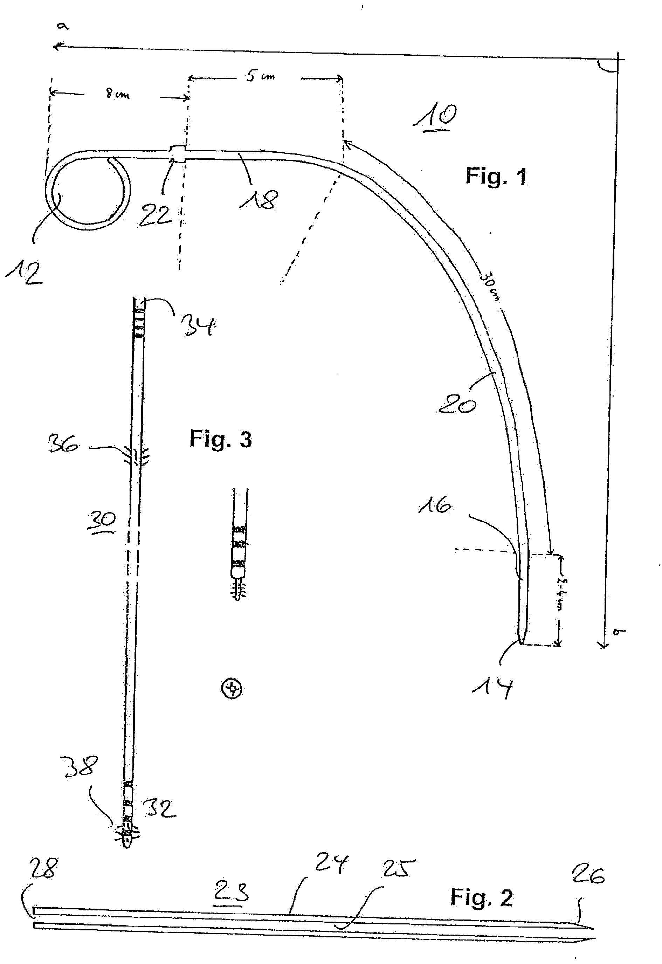

Die Fig. 1 und 2 beschreiben mit einem Ausführungsbeispiel des Dorns 10 (Fig. 1) bzw. der Hülse (Fig. 2) die wesentlichen Komponenten des chirurgischen Applikationswerkzeugs in der gezeigten Ausführungsform. Der Dorn ist, wie die Fig. 1 zeigt, realisiert als langgestreckt-gebogenes, aus einem zylindrischen Metallmaterial geformtes Werkzeug 10, welches einends einen gebogenen Griffabschnitt 12 (Segment A) und anderenends eine (abnehmbare) Eingriffsspitze 14 ausbildet, die endseitig eines geraden End- bzw. Eingriffssegments 16 (Segment 4) ausgebildet ist. Zwischen dem Griffabschnitt 12, für welchen das Dornmetall eines Außendurchmessers von 2 mm bis 5 mm, insbesondere 2,5 mm bis 3,5 mm, in der gezeigten Weise zu einer Schlaufe als Griffabschnitt für den extrakorporalen Zugriff gebogen ist, und dem Eingriffssegment 16 ist zunächst ein gerades Segment 18 mit einer Länge von ca. 5 cm, ein sich im Radius erweiterndes bogenförmiges Segment 20 vorgesehen, welches dann in das endseitige (distale) gerade Segment 16 übergeht. Ein Biegeradius des gebogenen Segments 30 bewegt sich zwischen ca. 40 cm und ca. 80 cm in Richtung auf das distale Ende.

Die Fig. 2 verdeutlicht die erfindungsgemäße Hülse 23, welche, realisiert aus einem biegesteifen, transparenten Kunststoffmaterial, mit einer Wandstärke von 2mm, auf die Segmente 18, 20, 16 des Dorns 10 aufschiebbar ist und, abgestützt von einem Widerlagerabschnitt 22, durch Angreifen an den Griffabschnitt 12 und Einführen in eine geeignet im Bauchdeckenbereich vorgesehene Körperöffnung in den Körper, entlang der Beckeninnenwand und bis zum Beckenboden bzw. den dort vorgesehenen pelvinen Nerven bzw. Nervenwurzeln gebracht werden kann; in einem Implantationszustand würde die Spitze 14 dann den konkreten Bereich im Beckeninneren markieren, an welchem die (später einzusetzende) Drahtelektrode mit ihrem Nervenkontaktabschnitt platziert werden kann.

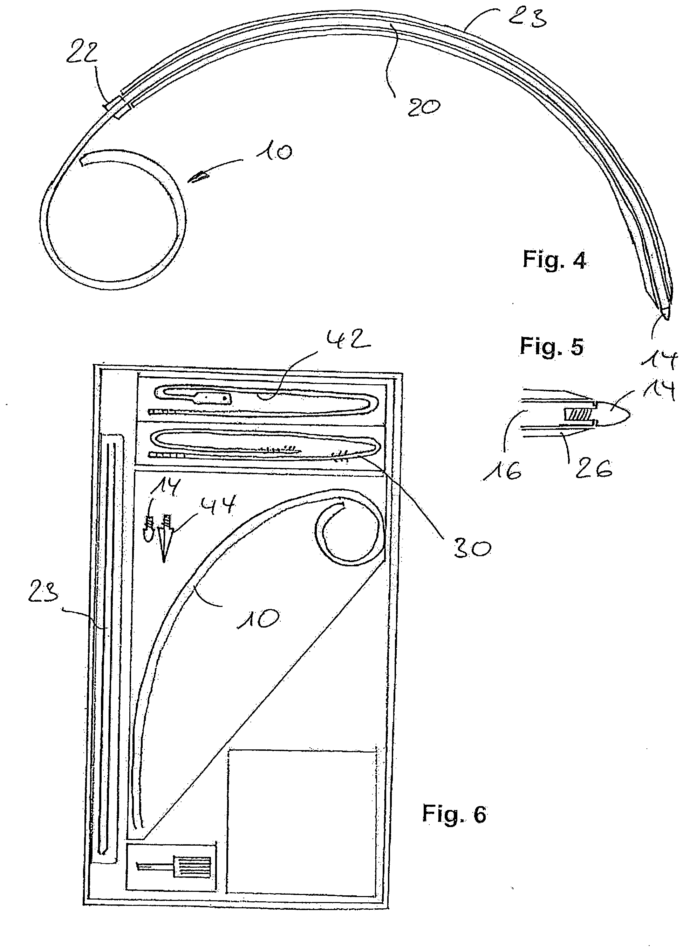

Wie die Fig. 2 verdeutlicht, ist der schlauchartig-ianggestreckte Schaftbereich 24 der Hülse 23 in Richtung auf das distale Ende 26 konisch zulaufend ausgebildet, wobei in bevorzugter Ausführungsform, vgl. die Spitzendarstellung der Fig. 4 im montierten Zustand der Hülse auf dem Dorn 10, ein spitz zulaufender Konus der Hülse 23 bevorzugt stufenlos einen konischen Verlauf der Eingriffsspitze 14 fortsetzt, so dass insoweit in dieser Einführungskonfiguration für das Werkzeug keine Gewebe- oder Gefäßbeschädigungen beim Einführen zu befürchten sind.

Die Fig. 4, insoweit diese Einführungskonfiguration beschreibend, verdeutlicht, dass das elastische Hülsenmaterial 24 der Hülse 23 dem (segmentweise) geraden bzw. bogenförmigen Verlauf des Dorns folgt und insoweit eine einfach handhabbare und positionierbare Werkzeugkonfiguration vorliegt. Gleichermaßen ist der Werkstoff für den Hülsenmantel 24 so ausgestaltet, dass dieser nicht nur mantelseitig glatt ist (insoweit wiederum günstig für ein reibungs- und ruptionfreies Gleiten bzw. Fortbewegen bei der Werkzeugeinführung und -bewegung im Körper), auch ist das Material derart biegesteif ausgestaltet, dass die Dornform (Fig. 1 bzw. Fig. 4) selbst dann erhalten bleibt, wenn nach dem Erreichen des Implantationsortes durch die Eingriffspitze 14 der Dorn 10 durch Herausziehen entfernt wird und die Hülse 23 in diesem Betriebs- bzw. Operationsstadium im Körper verbleibt (typischerweise sind die Geometrien so gewählt, dass im dem Segment 18 entsprechenden Längenabschnitt der Hülse diese aus dem Körper heraustritt und in einem dem distalen Bereich 26 entgegengesetzten Öffnungsbereich 28 nach entferntem Dorn 10 eine Einführungsöffnung für einen Elektrodendraht 30 (Fig. 3) anbietet).

Konkret im Gebrauch würde dieser Elektrodendraht einer typischen Länge zwischen 50 cm und 70 cm dann mit seinem distalen Nervenkontaktabschnitt 32 (die Fig. 3 zeigt insoweit zwei Varianten, 32 sowie, gesondert vergrößert herausgehoben, die alternative Ausgestaltung) in die Hülse 24 eingeschoben und im hohlzylindrischen Hülseninnenraum 25 geführt. Bevorzugt unter visuell-optischer Kontrolle eines geeignet über einen separaten Körpereingang an den Implantationsort gebrachtes Endoskops wird in der Bedienung des Systems der Elektrodendraht mit dem distalen Ende soweit vorgeschoben, bis er aus dem distalen Hülsenende 26 heraustritt; die Hülse hat im günstigen Fall ihre Position, in welche sie durch den Dorn 10 gebracht wurde, nicht verändert, so dass dann der Nervenkontaktabschnitt bereits an der vorgesehen Nervenkontaktposition (Implantationsposition) am gewünschten Nerv liegt. Ggf. hat der Operateur die Möglichkeit, durch geringe manuelle Betätigung der Hülse 23 vom extrakorporalen Hülsenende 28 aus Feinjustagen am Eingriffsort unter endoskopischer Kontrolle vorzunehmen.

Die einen Außendurchmesser von ca. 1,8 mm aufweisende und im gezeigten Ausführungsbeispiel 4-polig (Bezugszeichen 32) bzw. 3-polig (Bezugszeichen) ausgestaltete Mehrpol-Elektrode ist an einem dem distalen Ende 32 entgegengesetzten Anschlussabschnitt 34 in ansonsten bekanter Weise durch Peripherieelektronik (etwa eine Herzschrittmacherelektronik) o.dgl. implantierbaren elektrischen Impulsgeber) mechanisch und elektrisch kontaktierbar, wobei die jeweiligen, in der Fig. 3 distal gezeigten mantelseitigen Kontaktflächen über geeignete Lizenstrukturen im Inneren der Drahtelektrode geführt und im Endbereich 34 kontaktierbar sind. Zusätzlich weist der Elektrodendraht 30 Widerhaken- bzw. Verriegelungsmittel in Form von mantelseitig ansitzenden, radial in Richtung auf das proximale Ende 34 gerichteten Flügeln 36 auf, welche, um den Umfang des Manteldrahtes herum verteilt, so angeordnet bzw. (bevorzugt einstückig) befestigt sind, dass diese während des Verschiebens (Gleitens) im Hülsen-Inneraum 25 nah am Elektrodenmantel anliegen und insoweit einen einfachen, kraftarmen Vorschub ermöglichen, jedoch in einem freigelegten Zustand im Körper (insbesondere nach dem Entfernen der Hülse 23) nach dem die Elektrode 30 platziert ist, durch radiales Aufweiten (Spreizen) und/oder in der Art einer Widerhakenstruktur eine Sperrwirkung gegen Zugkräfte am Draht entfalten, welche in Richtung des proximalen Endes 34 gerichtet sind. Mit anderen Worten, die sich flügelartig entfaltenden Widerhaken- bzw. Verriegelungsmittel 36 sorgen vorteilhaft für eine Verankerung der Drahtelektrode 30 im Körper, so dass insbesondere Körperbewegungen oder aber eine unabsichtliche Zugwirkung auf die Elektrode 30 keine unbeabsichtigte Verschiebung oder gar Extraktion der Elektrode von ihrem Eingriffsort bewirken.

Denselben Zweck verfolgen am distalen Eingriffsende vorgesehene, analog flügelartig ausgebildete Widerhakenabschnitte 38 (bei einem, in der Variante am distalen Ende schmaleren Spitzenverlauf der Elektrode, insoweit in diesem Spitzenbereich ausgebildeten Widerhaken), so dass zusätzlich und bereits ab dem Zeitpunkt des Heraustretens der Elektrode 30 aus dem distalen Ende der Hülse 26 eine gewisse Sperrwirkung bzw. Sicherung gegen unbeabsichtigtes Zurückziehen geboten ist. Gerade die distalen Verankerungen (Widerhakenmittel) 38 bewirken dann auch vorteilhaft, das nach dem vollständigen Einschieben der Elektrode 30 erfolgenden manuellen Herausziehen der Hülse 23 nicht etwa ein Mitnehmen des Drahtes erfolgt, dieser verbleibt in seiner gewünschten implantierten Position, behält seinen (wiederum durch die vorbestimmte Krümmung der Hülse bzw. dem Dorn) vorbestimmten Implantations-Verlauf und ist im Idealfall vollständig unbeeinträchtigt von der Entfernung der Hülse 23, so dass am Ende dieses Bedienungsschrittes des erfindungsgemäßen chirurgischen Applikationswerkzeugs die Drahtelektrode 30 als alleinige implantierte Baugruppe im Körper verbleibt.

Im weiteren Betrieb würde nunmehr über das kontaktseitige, proximale Ende 34 der implantierten Elektrode 30 entweder zunächst ein Elektroden-Funktionstest (über extrakorporal angeschlossene Signalerzeugungsmittel und entsprechende Beobachtung der Nervenreaktion) erfolgen, oder es würde bereits, unmittelbar an das Ende 34 anschließbar oder mittels eines zusätzlich möglichen Verbindungsdrahtes 42 (Fig. 6) vorsehbar, der (nicht gezeigte) Impulsgeber geeignet konnektiert und dann seinerseits unter der Haut des Patienten geeignet platziert werden; die vorteilhafte Verlängerung 42, in Verbindung mit einer (erneuten) Verwendung des chirurgischen Applikationswerkzeugs bestehend aus Dorn 10 und Hülse 23 zum Verlegen des Verlängerungsdrahtes 42 aus der Endposition des Drahtendes 34 in eine andere Körperposition ermöglicht größere Flexibilität in der Implantation, zusätzlich erfährt der betroffene Patient an der für die Einführung des Applikationswerkzeugs notwendigen Öffnung eine geringere Belastung bzw. Infektionsgefahr durch den zu implantierenden Signalgenerator. Im Idealfall kann diese Körperöffnung vollständig verschlossen werden und ohne weitere Belastung (mit Ausnahme dann der Verbindung zwischen den Leitungen 30, 42) ausheilen.

Besonders günstig ist es, wenn das System in der in Fig. 6 schematisch gezeigten Art mit den benötigten Komponenten in der Art einer einfach zugreifbaren Packung bzw. eines Kitts bereitgestellt wird. Neben den diskutierten Hauptkomponenten Dorn 10 und Hülse 23 zeigt dieses Kitt insoweit auch ein alternatives Eingriffspitzenelement 44, welches, austauschbar mittels der Schraubung o.dgl. gegen ein konisch zulaufendes, stumpfes Element 14 (Fig. 5) besonders geeignet ist, in dem fakultativ beschriebenen zweiten Verwendungs- bzw. Behandlungsschritt für das Verlängerungskabel 42 dessen Verlauf (typischerweise nahe der Haut im Bauchbereich) auszuformen.

Die vorliegende Erfindung ist nicht auf die gezeigten Ausführungsbeispiele beschränkt; so ist es insbesondere möglich und bevorzugt, nicht nur den Dorn bzw. die Hülse geeignet durch Biegen bzw. Formgestaltung an jeweilige orthopädisch-geometrische Bedingungen in einem Beckeninnenbereich eines jeweiligen Patienten anzupassen, auch ermöglicht etwa die Materialwahl für den Dorn ein Nachbiegen bzw. ein zusätzliches Einbringen von im Einzellfall sinnvollen oder vorteilhaften weiteren Deformationen des Werkzeugverlaufs, welcher gemäß der Erfindung insoweit den Einführungs- und Implantationsverlauf für die Drahtelektrode vorgibt.

## Patentansprüche

1. Chirurgisches Applikationswerkzeug zum Implantieren eines Elektrodendrahts an Nerven und Nervenwurzeln in einen Beckeninnenbereich eines menschlichen Körpers mit einem eine Eingriffsspitze (14), einen Schaftabschnitt (18, 20) sowie einen zur extrakorporalen Handhabung ausgebildeten Griffabschnitt (12) aufweisenden Dorn
und einer sich zumindest entlang des Schaftabschnitts erstreckenden, auf diesem führbaren und in einem in den Körper eingeführten Zustand vom Dorn lösbaren Hülse (23),
die zum Einsetzen und Führen des Elektrodendrahts durch einen Hülsen-Innenraum bei entferntem Dorn ausgebildet ist,
wobei der Schaftabschnitt zumindest segmentweise bogenförmig und plastisch verformbar ausgebildet ist,
wobei der Griffabschnitt zum manuellen Drehen und/oder Verschwenken des Schaftabschnitts in dem in den Körper eingeführten Zustand ausgebildet ist,
**dadurch gekennzeichnet, dass**
ein **nicht kreisbogenförmiger, sich in Richtung auf die Eingriffsspitze verlängernder** Biegeradius eines bogenförmigen Segments des **zum Führen retroperitoneal entlang der Wand der Bauchdecke des menschlichen Körpers ausgebildeten und vorgesehenen** Dorns zwischen 15 cm und 70 cm, insbesondere zwischen 20 cm und 50 cm beträgt, dass der Dorn zwischen der Eingriffsspitze und dem mindestens einen bogenförmigen Segment ein eingriffsseitiges gerades Segment einer Länge zwischen 1 cm und 5 cm aufweist, bevorzugt zwischen 2 cm und 4 cm, und dass die Hülse aus einem zumindest **eine endoskopische Überwachung des Führens des Elektrodendrahts gestattenden, zumindest abschnittsweise transparenten,** biegesteifen Werkstoff realisiert ist.

2. Werkzeug nach Anspruch 1, **dadurch gekennzeichnet, dass** der Dorn aus einem Metallmaterial einer Länge von 25 cm bis 70 cm, bevorzugt 30 cm bis 60 cm, weiter bevorzugt zwischen 40 cm und 50 cm, realisiert ist.

3. Werkzeug nach Anspruch 1 oder 2, **dadurch gekennzeichnet, dass** ein bevorzugt konstanter Außendurchmesser des Dorns zwischen 2 mm und 6 mm, insbesondere zwischen 3 mm und 5 mm, beträgt und weiter bevorzugt an eine lichte Innenweite des Hülsen-Innenraums spielbehaftet angepasst ist.

4. Werkzeug nach einem der Ansprüche 1 bis 3, **dadurch gekennzeichnet, dass** der eine Krümmung oder Abwinkelung aufweisende Griffabschnitt in der Ebene des bogenförmigen Segments liegt.

5. Werkzeug nach einem der Ansprüche 1 bis 4, **dadurch gekennzeichnet, dass** der Dorn zwischen dem Griffabschnitt und dem mindestens einen bogenförmigen Segment ein griffseitiges gerades Segment, bevorzugt einer Länge zwischen 3 cm und 7 cm, weiter bevorzugt zwischen 4 cm und 6 cm, aufweist.

6. Werkzeug nach einem der Ansprüche 1 bis 5, **dadurch gekennzeichnet, dass** das bogenförmige Segment eine von einem Kreisbogen verschiedene, sich in Richtung auf die Eingriffsspitze radial vergrößernde Bogenform und/oder einen S-förmigen Bogenverlauf aufweist.

7. Werkzeug nach einem der Ansprüche 1 bis 6, **dadurch gekennzeichnet, dass** der Dorn ein lös- und/oder austauschbares Eingriffsspitzenelement (14) aufweist, insbesondere konisch zulaufend mit einem stumpfen Ende ausgebildet und gegen ein spitz zulaufend ausgebildetes Eingriffsspitzenelement austauschbar realisiert ist.

## Claims

1. Applicateur chirurgical d'implantation d'un fil d'electrode aux nerfs et aux racines nerveuses dans un intérieur de pelvis d'un corps humain, ayant une broche comportant une pointe de contact (14), une zone de manche (18, 20) ainsi qu'une zone de poignée (12) configurée pour une manipulation extracorporelle
et ayant une douille (23) s'etendant au moins le long de la zone de manche et pouvant être guidée sur celle-ci et étant démontable de la broche en état introduit dans le corps,
la douille étant configurée pour placer et guider le fil d'electrode à travers un intérieur de douille lorsque la broche a été enlevée,
ladite zone de manche étant configurée à être arquée au moins par segments et à être susceptible de subir une déformation plastique, ladite zone de poignée étant configurée pour tourner et/ou pivoter la zone de manche, à la main, en état introduit dans le corps, **caractérisé en ce qu'**
un rayon de courbure d'un segment arqué de la broche configurée et prévue pour un guidage rétropéritonéal le long de la paroi abdominale du corps humain, le rayon étant non en arc de cercle et étant allongé dans la direction de la pointe de contact, est compris entre 15 cm et 70 cm, en particulier entre 20 cm et 50 cm,
**en ce que** la broche, entre la pointe de contact et l'au moins un segment arqué, comporte un segment droit du côté de contact ayant une longueur entre 1 cm et 5 cm, de préférence entre 2 cm et 4 cm,
et **en ce que** la douille est réalisée d'une substance résistante à la flexion permettant au moins une supervision endoscopique du guidage du fil d'electrode et étant transparente au moins par zones.

2. Applicateur selon la revendication 1, **caractérisé en ce que** la broche est réalisée d'un matériau métallique ayant une longueur de 25 cm à 70 cm, de préférence 30 cm à 60 cm, de préférence encore entre 40 cm et 50 cm.

3. Applicateur selon la revendication 1 ou 2, **caractérisé en ce qu'**un diamètre extérieur de la broche, le diamètre étant de préférence constant, est compris entre 2 mm et 6 mm, en particulier entre 3 mm et 5 mm, et de préférence encore est adapté à un diamètre intérieur de l'intérieur de douille avec jeu.

4. Applicateur selon l'une quelconque des revendications 1 à 3, **caractérisé en ce que** la zone de poignée comportant une courbe ou une pliure est située dans le plan du segment arqué.

5. Applicateur l'une quelconque des revendications 1 à 4, **caractérisé en ce que** la broche, entre la zone de poignée et l'au moins un segment arqué, comporte un segment droit du côté de contact, de préférence ayant une longueur entre 3 cm et 7 cm, de préférence encore entre 4 cm et 6 cm.

6. Applicateur selon l'une quelconque des revendications 1 à 5, **caractérisé en ce que** le segment arqué comporte une forme d'arc différent d'un arc de cercle et s'agrandissant dans la direction de la pointe de contact et/ou un trajet d'arc en forme de S.

7. Applicateur selon l'une quelconque des revendications 1 à 6, **caractérisé en ce que** la broche comporte un élément (14) de pointe de contact démontable et/ou remplaçable, en particulier étant configuré à se terminer en cône ayant une extrémité épointée et étant réalisé à être remplaçable par un élément de pointe de contact configuré à se terminer en pointe.

## Revendications

1. A surgical applicator tool for
implanting an electrode wire to nerves and nerve roots into a pelvis interior of a human body,
having a mandrel featuring an engagement tip (14), a stem portion (18, 20) as well as a holding portion (12) embodied for extracorporeal handling
and having a sleeve (23) that extends at least along the stem portion, that is guidable on said stem portion and that is détachable from the mandrel when inserted in the body,
which sleeve is embodied for introducing and guiding the electrode wire through a sleeve interior when the mandrel has been removed,
the stem portion being embodied so as to be curved at least in segments and so as to be plastically deformable,
the holding portion being embodied for manually turning and/or pivoting the stem portion when inserted in the body,
**characterised in that**
a bending radius of a curved segment of the mandrel embodied and designated for retroperitoneal guidance along the abdominal wall of the human body, said bending radius not having the shape of a circular arc and being elongated in the direction of the engagement tip, amounts to between 15 and 70 cm, in particular between 20 and 50 cm,
**in that** the mandrel, between the engagement tip and the at least one curved segment, features a straight segment on the engagement side having a length between 1 cm and 5 cm, preferably between 2 cm and 4 cm,
and **in that** the sleeve is realised from a bending-resistant material allowing at least endoscopically monitoring the guiding of the electrode wire and being transparent at least in some portions.

2. The tool according to claim 1, **characterised in that** the mandrel is realised from a metal material having a length of 25 cm to 70 cm, preferably 30 cm to 60 cm, more preferably between 40 cm and 50 cm.

3. The tool according to claim 1 or 2, **characterised in that** an outer diameter of the mandrel, preferably being constant, amounts to between 2 mm and 6 mm, in particular between 3 mm and 5 mm, and is more preferably adapted to an inner width of the sleeve interior with a clearance.

4. The tool according to any one of the claims 1 to 3, **characterised in that** the holding portion featuring a curve or an angular offset is located in the plane of the curved segment.

5. The tool according to any one of the claims 1 to 4, **characterised in that** the mandrel, between the holding portion and the at least one curved segment, features a straight segment on the engagement side, preferably having a length between 3 cm and 7 cm, more preferably between 4 cm and 6 cm.

6. The tool according to any one of the claims 1 to 5, **characterised in that** the curved segment features a curvature other than a circular arc and radially increasing in the direction of the engagement tip and/or an S-shaped curve course.

7. The tool according to any one of the claims 1 to 6, **characterised in that** the mandrel features a detachable and/or replaceable engagement tip element (14), in particular being embodied so as to be tapered having a butt and being realised so as to be replaceable with an engagement tip element embodied so as to be pointed.
